# EUROPÄISCHE PATENTSCHRIFT

(11) **EP 2 934 362 B1**
(45) Veröffentlichungstag und Bekanntmachung des Hinweises auf die Patenterteilung: **01.05.2019**
(21) Anmeldenummer: 13828772.7
(22) Anmeldetag: 12.12.2013
(51) Int. Cl.: A61B 34/30, A61B 90/11

(54) **AKTIVE POSITIONIEREINRICHTUNG EINES CHIRURGISCHEN INSTRUMENTS UND EIN DIESE UMFASSENDES CHIRURGISCHES ROBOTERSYSTEM**
ACTIVE POSITIONING ARRANGEMENT OF A SURGICAL INSTRUMENT AND ROBOTIC SURGICAL SYSTEM COMPRISING SAME
DISPOSITIF DE POSITIONNEMENT ACTIF D'UN INSTRUMENT CHIRURGICAL ET SYSTÈME ROBOTIQUE COMPORTANT CE DISPOSITIF

(30) Priorität: 20.12.2012 DE 102012025101
(43) Veröffentlichungstag der Anmeldung: 28.10.2015
(73) Patentinhaber: avateramedical GmbH, 07745 Jena (DE)
(72) Erfinder: VON GRÜNBERG, Hubertus, 30625 Hannover (DE)
(74) Vertreter: Patentanwälte Geyer, Fehners & Partner mbB
(86) Internationale Anmeldenummer: PCT/DE2013/000806
(87) Internationale Veröffentlichungsnummer: WO 2014/094719

(56) Entgegenhaltungen:
- EP-A1- 2 324 790
- WO-A1-2006/035143
- DE-A1-102009 012 987
- US-A1- 2010 234 856
- US-A1- 2011 152 717
- US-A1- 2011 319 913
- US-A1- 2013 317 521

## Beschreibung

Die vorliegende Erfindung betrifft eine aktive Positioniereinrichtung eines chirurgischen Instruments und ein chirurgisches Robotersystem bzw. Telemanipulator für die minimal-invasive Chirurgie und insbesondere der Laparoskopie.

Robotersysteme oder auch Telemanipulatoren für die minimal-invasive Chirurgie, insbesondere für die laparoskopische Chirurgie, ersetzen die vom Chirurgen üblicherweise manuell geführten Operations-Instrumente, wie beispielsweise chirurgische Instrumente, Endoskop bzw. Kamera, durch eine motorische Positionierung. Die einzusetzenden Operations-Instrumente werden über einen bzw. mehrere Trokare in das Innere eines Patienten geführt. Als Trokar wird ein Instrument bezeichnet, mit dessen Hilfe der Chirurg in der minimal-invasiven Chirurgie ein Zugang zu der Körperhöhle des Patienten (üblicherweise des Bauchraumes oder des Brustraumes) schafft, wobei der Zugang durch ein Rohr, einen sog. Tubus, offengehalten wird. Die in dem Robotersystem vorgesehene Bewegungsmechanik und Steuerungslogik ermöglicht die Bewegung der Operations-Instrumente um einen Pivotalpunkt herum in 2 Freiheitsgraden (x, y) sowie eine translatorische Bewegung der Operations-Instrumente entlang der Instrumentenachse (z). Als Pivotalpunkt wird der invariante Punkt der Bewegung in 2 Freiheitsgraden (x, y) bezeichnet. Dieser Pivotalpunkt liegt idealerweise im Durchstoßpunkt des Trokars durch die Bauchdecke des Patienten. Die Steuerungslogik eines Robotersystems muss den Pivotalpunkt kennen bzw. der Pivotalpunkt muss durch die konstruktive Ausführung der Bewegungsmechanik definiert sein, um eine Bewegung des Operations-Instrumentes so zu begrenzen, dass die biomechanische Belastung des Gewebes um den Trokar herum möglichst gering ist.

Aus dem Stand der Technik bekannte Robotersysteme basieren auf Roboterarmen zur passiven Vorpositionierung und aktiven Bewegung eines Operations-Instrumentes. Eine Lösung mit Roboterarmen aus dem Stand der Technik, die eine passive Vorpositionierung und eine aktive Bewegung der Operations-Instrumente rund um den Pivotalpunkt realisieren, benötigen einerseits einem großen Bauraum und andererseits können die Bewegungsabläufen der Roboterarmen zu einer Kollision führen.

Während eines minimal-invasiven chirurgischen Eingriffs werden mindestens zwei, in der Regel drei bis vier chirurgische Instrumente, wie Greifer, Schere, Nadelhalter, Sezierer), sowie eine Kamera bzw. ein Endoskop verwendet, die jeweils über einen gesonderten Trokar in das Körperinnere des Patienten geführt werden. Das bedeutet, dass für jedes eingesetzte Operations-Instrument ein Roboterarm vorhanden ist, der die passive Vorpositionierung und die aktive Bewegung des Instruments steuert.

US2011/0319913 offenbart ein Gerät zur Implantation mit einer Plattform und einem Parallelroboter.

Nachteil der Lösungen aus dem Stand der Technik ist, dass die Position des Patienten vor Beginn der Operation fixiert werden muss und eine Umlagerung des Patienten während der Operation nahezu nicht möglich ist.

Ein weiterer, bereits erwähnter, Nachteil ist der große Bauraum, den die bekannten Robotersysteme erfordern.

Aufgabe der vorliegenden Erfindung ist es daher, eine aktive Positioniereinrichtung eines chirurgischen Instruments und ein chirurgisches Robotersystem bereitzustellen, die bzw. das eine hohe Variabilität vorsieht und nur einen geringen Bauraum benötigt bzw. kleiner und leichter in seiner Ausführung ist.

Eine weitere Aufgabe der vorliegenden Erfindung ist es, ein Robotersystem bereitzustellen, das eine Umlagerung des Patienten während einer Operation ermöglicht, insbesondere ohne dabei die Bewegungsfreiheit des chirurgischen Instruments nach der Umlagerung einzuschränken.

Diese Aufgaben werden durch die vorliegende Erfindung gemäß Anspruch 1 durch eine aktive Positioniereinrichtung eines chirurgischen Instruments und gemäß Anspruch 9 durch ein chirurgisches Robotersystem gelöst.

Ein Gegenstand der vorliegenden Erfindung betrifft eine aktive Positioniereinrichtung eines chirurgischen Instruments zur Verwendung an einem Roboterarm nach Anspruch 1. Bevorzugte Ausführungsformen sind in den abhängigen Ansprüchen beschrieben.

In einer bevorzugten Ausführungsform ist das Ausgleichselement in seiner Geometrie derart variierbar, dass ein frei wählbarer Winkel sowohl in x- als auch in y-Richtung zwischen der Porteinrichtung und der Führungseinrichtung im Hinblick auf die zueinander kongruente Ausgangslage einstellbar ist, wobei das Ausgleichselement insbesondere aus einem elastischen Material ausgebildet ist.

Erfindungsgemäß weist die Verstelleinrichtung zumindest zwei ansteuerbare Aktuatoren auf, welche insbesondere als zueinander orthogonal angeordnete Stellantriebe ausgeführt sind, wobei ein Kugelhebelmechanismus zwischen der Führungseinrichtung und der Trägerplatte oder der Porteinrichtung derart vorgesehen ist, dass durch die Stellantriebe mittels des Kugelhebelmechanismus die Führungseinrichtung gegenüber der Ausgangslage in x-Richtung und y-Richtung unabhängig voneinander positionierbar ist.

Bei einer ebenfalls bevorzugten Ausführungsform ist an der Führungseinrichtung eine Translationsverstelleinrichtung vorgesehen, die mit dem chirurgischen Instrument derart verbunden ist, dass der Schaft des chirurgischen Instruments in z-Richtung bewegbar ist. Bevorzugt bewegt die Translationsverstelleinrichtung mittels eines Teleskopsystems 20 und/oder Seilzugsystems den Schaft des chirurgischen Instruments in z-Richtung.

In einer ferner bevorzugten Ausführungsform ist an dem chirurgischen Instrument eine Instrumentenantriebseinheit vorgesehen, welche einen Rotationsaktuator umfasst, durch den der Schaft des chirurgischen Instruments gegenüber der Ausgangslage um die z-Richtung drehbar variiert wird. Bevorzugt weist die Instrumentenantriebseinheit drei Instrumentenaktuatoren auf, durch welche die am distalen Ende angebrachte Wirkeinheit des chirurgischen Instruments in drei weiteren Freiheitsgraden variierbar ist. Insbesondere bevorzugt ist die Instrumentenantriebseinheit über eine Halteeinrichtung an dem proximalen Ende des Teleskopsystems angeordnet.

Ein weiterer Gegenstand der vorliegenden Erfindung betrifft ein chirurgisches Robotersystem zur Durchführung von operativen Eingriffen am menschlichen Körper nach Anspruch 9.

In einer bevorzugten Ausführungsform des Robotersystems ist das Ausgleichselement in seiner Geometrie derart variierbar, dass ein frei wählbarer Winkel sowohl in x- als auch in y-Richtung zwischen der Porteinrichtung und der Führungseinrichtung im Hinblick auf die zueinander kongruente Ausgangslage einstellbar ist, wobei das Ausgleichselement insbesondere aus einem elastischen Material ausgebildet ist.

Weitere vorteilhafte Ausgestaltungen des erfindungsgemäßen chirurgischen Robotersystems ergeben sich aus den Unteransprüchen analog zu der aktiven Positioniereinrichtung für ein chirurgisches Instrument. Dies ergibt sich insbesondere dadurch, dass die erfindungsgemäße aktive Positioniereinrichtung mit einem Robotersystem kombiniert bzw. nachgerüstet werden kann.

Erfindungsgemäß können die Begriffe Robotersystem und Telemanipulator synonym verwendet werden.

Die vorliegende Erfindung wird rein beispielhaft durch die beigefügten Figuren realisiert. Es zeigt:
Figur 1 eine schematische Ansicht der erfindungsgemäßen aktiven Positioniereinrichtung eines chirurgischen Instruments, welche an einem Roboterarm angebracht ist;
Figur 2 eine schematische Teilansicht der erfindungsgemäßen aktiven Positioniereinrichtung mit Anschlussmöglichkeit zur Einleitung von Insufflationsgas, in der Regel CO2;
Figur 3 eine Draufsicht der aktiven Positioniereinrichtung nach Figur 1;
Figur 4 eine schematische Ansicht eines Teils des erfindungsgemäßen chirurgischen Robotersystems während einer Operation am menschlichen Körper;
Figur 5 eine schematische Ansicht eines Roboterarms gemäß der Erfindung;
Figur 6 eine schematische Ansicht eines chirurgischen Instruments, welches Bestandteil der Erfindung sein kann;
Figur 7 eine schematische Ansicht eines Robotersystems mit einem Roboterarm und aktiven Positioniereinrichtungen gemäß der Erfindung; und
Figur 8 eine schematische Ansicht eines Robotersystems mit 4 Roboterarmen und aktiven Positioniereinrichtungen gemäß der Erfindung.
Figur 9 eine schematische Ansicht der erfindungsgemäßen aktiven Positioniereinrichtung für zwei chirurgische Instrumente und einem gemeinsamen Port, welche an einem Roboterarm angebracht ist;
Figur 10 eine Draufsicht der aktiven Positioniereinrichtung nach Figur 9

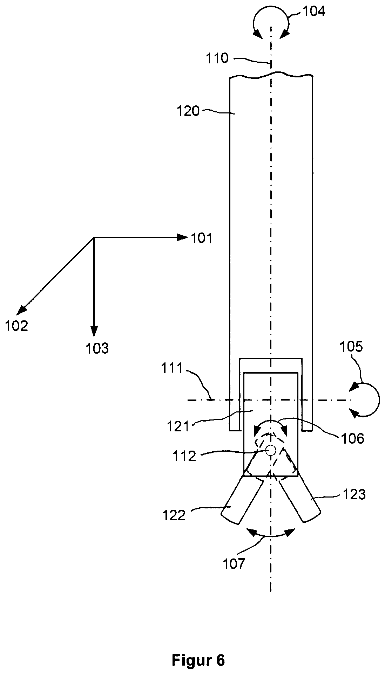

Die vorliegende Erfindung betrifft gemäß einem Aspekt ein chirurgisches Robotersystem bzw. einen Telemanipulator, bei dem eine passive Vorpositionierung des Trokars bzw. der aktiven Positioniereinrichtung mit einer aktiven Steuerung bzw. Motorisierung des Trokars zur Bewegung eines Operations-Instrumentes kombiniert wird. Ein solcher erfindungsgemäßer "aktiver Trokar" kann das Operations-Instrument um den Pivotalpunkt zumindest in 2 Freiheitsgraden (Richtung 101 und 102) bewegen, wie in Figur 6 dargestellt ist. Erfindungsgemäß weisen die Operations-Instrumente insgesamt 7 Freiheitsgrade auf: 3 Freiheitsgrade (Freiheitsgrade 101, 102, und 103 gemäß Figur 6) werden durch eine motorische Kopplung des in den Trokar eingesetzten Operations-Instruments mit entsprechenden Antriebseinheiten realisiert, weitere 4 Freiheitsgrade (Freiheitsgrade 104, 105, 106 und 107 gemäß Figur 6) werden durch eine Antriebseinheit am Ende eines jeden eingesetzten Operations-Instrumentes realisiert.

Da der Pivotalpunkt durch den aktiven Trokar selbst definiert wird, wird die Lage des der Pivotalpunktes vor Beginn der Operation über eine Vorpositionierung des aktiven Trokars festgelegt. Eine Umlagerung des Patienten nach dem Beginn des chirurgischen Eingriffs ist damit möglich, da der Pivotalpunkt mit den erfindungsgemäßen Positioniereinrichtungen bzw. aktiven Trokaren konstruktiv verbunden ist und bei einer Umlagerung gegenüber der aktiven Positioniereinrichtung beibehalten wird, d.h., dass der Pivotalpunkt gegenüber dem Instrument und der Trägerplatte sowie Führungseinrichtung stets beibehalten wird.

Weiterhin kann durch den Verzicht auf Roboterarme zur aktiven Positionierung der Instrumente das System deutlich kleiner und leichter ausgeführt werden. Damit ist ein einfacherer Transfer des Gesamtsystems, beispielsweise in einen anderen Operations-Saal, und damit eine höhere Flexibilität und Nutzungsauslastung möglich.

Die vorliegende Erfindung wird nachfolgend im Detail unter Bezugnahme auf die Figuren beschrieben:

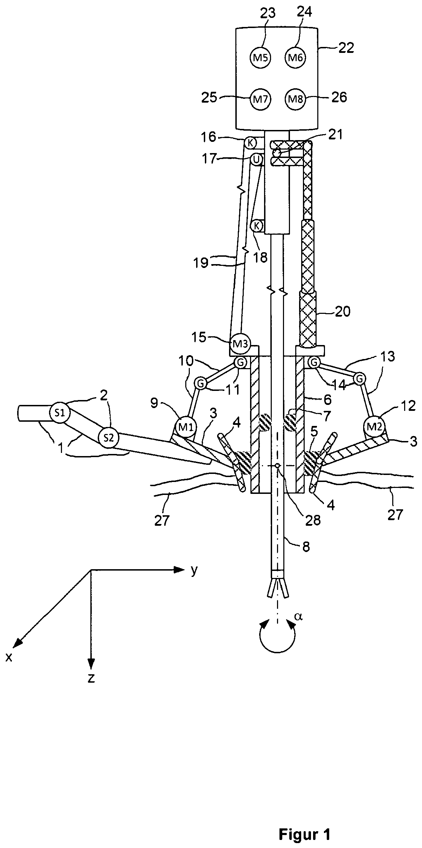

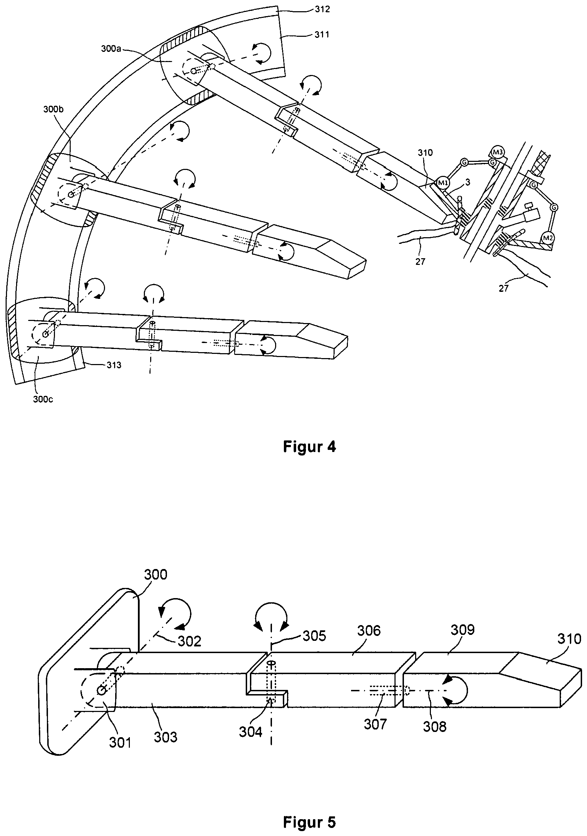

**Figur 1** zeigt eine erfindungsgemäße aktive Positioniereinrichtung eines chirurgischen Instruments, die an einem Roboterarm angebracht ist. Während eines minimal-invasiven, laparoskopischen Eingriffs kommen in der Regel 4 Operations-Instrumente zum Einsatz, hiervon 3 chirurgische Instrumente und 1 Kamera bzw. Endoskop, die über das Telemanipulatorsystem vom Operateur gesteuert werden. Erfindungsgemäß sind demnach vorzugsweise 4 Ausführungen eines aktiven Trokars bzw. einer aktiven Positioniereinrichtung im System vorhanden. Es versteht sich jedoch, dass auch Ausführungsformen mit 1 bis 3 oder mehr als 4 aktiven Trokaren unter den Schutzbereich der Erfindung fallen. Jeder aktive Trokar wird über einen Roboterarm 1, welche mit Gelenken 2 versehen sein kann gewichtskraftfrei gelagert. Dieser Halterungsmechanismus ist demnach für jeden aktiven Trokar vorzusehen. Alle Halterungsmechanismen können an einem gemeinsamen Träger (siehe Figur 4), oder an getrennten Trägern befestigt werden. Eine Befestigung an getrennten Trägern kann beispielsweise sinnvoll sein, wenn die Anordnung der Trokare für den chirurgischen Eingriff dies erfordert.

Mit dem Roboterarm 1 ist eine Trägerplatte 3 des aktiven Trokars fest verbunden. Diese Trägerplatte 3 ist wiederum fest mit einer Porteinrichtung 4 verbunden. Die Porteinrichtung 4 ist seinerseits über ein Ausgleichselement 5 mit der Führungseinrichtung 6 verbunden. Über das Ausgleichselement 5 ist eine Bewegung (Neigung) der Führungseinrichtung 6 gegenüber der Porteinrichtung 4 möglich. Durch diese Bewegung wird die pivotale Bewegung des chirurgischen Instruments 8 realisiert. Die Führungseinrichtung 6 nimmt das chirurgische Instrument 8 auf. Über einen Dichtungsring 7 erfolgt eine gasdichte Abdichtung des chirurgischen Instrumentes 8 gegenüber der Führungseinrichtung 6. In der Laparoskopie wird der Bauchraum durch Einleitung eines Gases (Kohlendioxid, CO2) "aufgepumpt", um dem Operateur mehr Bewegungsfreiheit für den eigentlichen chirurgischen Eingriff zu ermöglichen. Damit das Gas nicht entweichen kann, ist die Abdichtung 7 erforderlich.

Die Aktuatoren bzw. Stellantriebe 9, 12 sind orthogonal zueinander angeordnet. Über einen Kugelhebelmechanismus 10, 11 sowie 13, 14 werden Kräfte auf das obere Ende der Führungseinrichtung 6 ausgeübt, so dass diese sich relativ zur Porteinrichtung 4 in 2 Achsen (x, y) unabhängig voneinander bewegen lässt.

Ein weiterer Stellantrieb 15 ist am oberen Ende der Führungseinrichtung 6 angebracht. Über eine Stellantriebsmechanik bestehend aus Klemme 16, Umlenkrolle 17, Klemme 18 und entsprechende Seilzüge 19 wird die translatorische Bewegung des Instrumentes in z-Richtung realisiert.

Ein Teleskopsystem 20 ist über die Halteeinrichtung 21 mit einer Instrumentenantriebseinheit 22 derart verbunden, das eine Rotationsbewegung α des chirurgischen Instrumentes 8 um die z-Achse verhindert wird. Die Rotationsbewegung α des chirurgischen Instruments 8 wird durch einen Rotationsaktuator 23, welcher mit dem Schaft des chirurgischen Instruments 8 verbunden ist, realisiert. Die Instrumentenaktuatoren 24, 25 und 26 realisieren die Bewegungen des chirurgischen Instruments 8 in den Freiheitsgraden 105, 106 und 107, siehe Figur 6.

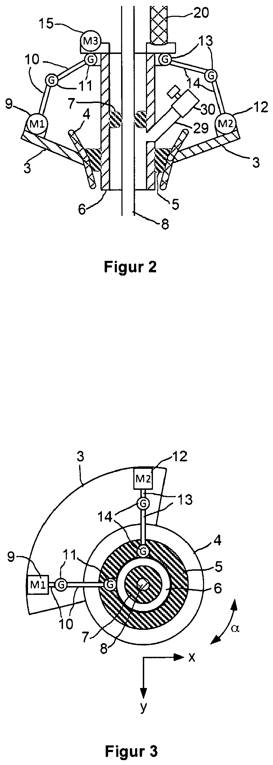

**Figur 2** zeigt eine erfindungsgemäße aktive Positioniereinrichtung wie in Figur 1, zusätzlich versehen mit einem Insufflations-Anschluss bestehend aus einer Zuführungsröhre (29), welche unterhalb der Dichtung (7) in den Hohlraum des Trokars (6) mündet sowie einer Anschluss- und Ventilbaugruppe (30). An die Anschluss- und Ventilbaugruppe wird zweckmäßigerweise ein Insufflations-Gerät angeschlossen. Dieses pumpt ein Gas, im Regelfall CO2 über die Ventilbaugruppe (30) und die damit verbundene Zuführungsröhre (29) in den Bauchraum des Patienten. Die Dichtung (7) verhindert ein ungewolltes Entweichen des Gases aus dem Bauchraum des Patienten in die Umgebung. Die Dichtung (7) ist zweckmäßigerweise so ausgeführt, dass diese bei vollständigen Entfernen des Instruments (8) aus dem Trokar (6) diesen gasdicht abschließt, d.h. auch bei entnommenen Instrument entweicht kein Insufflationsgas in die Umgebung.

**Figur 3** zeigt eine Draufsicht der aktiven Positioniereinrichtung gemäß Figur 1;

**Figur 4** zeigt eine schematische Ansicht eines Teils des erfindungsgemäßen chirurgischen Robotersystems während einer Operation am menschlichen Körper; die in Figur 5 detailliert beschriebenen Roboterarme werden durch Basishalterungen, hier exemplarisch 300a, 300b und 300c, derart gehaltert, das diese Basishalterungen in einer z.B. bogenförmig ausgeprägten Führungsschiene 311 in Verbindung mit 312 und 313 gehaltert werden und relativ zueinander unabhängig positioniert werden können. An der Koppelfläche 310 des Roboterarms wird die Aktive Positioniereinrichtung gemäß Figur 1 bzw. 2 an der Baugruppe 3 gehaltert. Die vorzugsweise bogenförmige Gestalt der Führung hat den Vorteil die Roboterarme gemäß der typischen Anatomie eines Patienten in einem Bogen oberhalb der Bauchdecke 27 vorzupositionieren.

**Figur 5** zeigt eine schematische Ansicht eines Roboterarms gemäß der Erfindung; Der Roboterarm besteht aus mehreren Gliedern 303, 306 und 309, welche über Gelenke miteinander so verbunden sind, das eine Anordnung der Koppelfläche 310 zu einer Aktiven Positioniereinrichtung gemäß Figur 1 bzw. 2 möglich ist. Der Roboterarm selbst ist über ein Gelenk 301, welches eine Schwenkbewegung um die Drehachse 302 um +/- 90° ermöglicht, an einer Basishalterung 300 befestigt. Das erste Teilglied 303 des Roboterarms führt zu einem weiteren Gelenk 304, welches eine Drehachse 305, vorzugsweise orthogonal zur Drehachse 302, aufweist. Über das Gelenk 304 ist ein weiteres Teilglied 306 des Roboterarms verbunden, welches über ein weiteres Gelenk 307 eine Schwenkbewegung 308 ermöglicht, vorzugsweise orthogonal zur Drehachse 302 und orthogonal zur Drehachse 305, angeordnet. Das dritte Teilglied 309 des Roboterarms weist an seinem distalen Ende eine Koppelfläche 310 auf, an welche durch Herstellung einer geeigneten kraft- und vorzugsweise auch formschlüssigen Verbindung die Aktive Positionierungseinrichtung gemäß Figur 1 bzw. 2 an der Baugruppe 3 befestigt werden kann. Die Gelenke 301, 304, 307 können sowohl aktiv, d.h. versehen mit Stellantrieben, als auch passiv ausgeführt sein. Die Gelenke 301, 304, 307 sind mit absoluten Positionsgebern versehen, so dass die Lage bzw. Orientierung der Roboterarme und der jeweils daran angekoppelten Aktiven Positioniereinrichtung im Raum bekannt ist. Die Signale der absoluten Positionsgeber können vorzugsweise in der Steuereinheit 202 so miteinander verrechnet werden, dass unter Kenntnis der Geometrie und der aktuellen Stelllage der Aktiven Positioniereinrichtung gemäß Figur 1 bzw. 2 eine drohende Kollision der verschiedenen Roboterarme untereinander bzw. die Kollision eines Roboterarmes mit einer Aktiven Positioniereinrichtung eines anderen Roboterarmes erkannt wird und über die Bedien- und Anzeigeeinheit 200 dem Anwender eine Kollisionswarnung ausgegeben werden kann. In einer weiteren Ausführungsform kann die Steuereinheit 202 die potentielle Kollision der verschiedenen Roboterarme untereinander, bzw. die Kollision eines Roboterarmes mit einer Aktiven Positioniereinrichtung eines anderen Roboterarmes durch eine Änderung des durch die Bedien- und Anzeigeeinheit 200 vorgegebenen Stellbefehls aktiv vermeiden. In einer passiven Ausführung sind die Gelenke 301, 304, 307 vorzugsweise mit einer Einrichtung gegen unbeabsichtigtes Verstellen der Gelenkposition gesichert.

**Figur 6** zeigt ein chirurgischen Instruments als einen möglichen Bestandteil der Erfindung. Insgesamt weist die Anordnung 7 Freiheitsgrade auf. Diese werden realisiert durch eine translatorische Bewegung des Instrumentenschaftes (120) in x-Richtung (101) und y-Richtung (102). Diese Bewegungen bewirken ein Verkippen des Instrumentes um den Pivotpunkt. Weiterhin kann der Instrumentenschaft (120) in z-Richtung (103) bewegt werden. Der Instrumentenschaft (120) kann um seine eigene Instrumentenachse (110) in der Bewegungsrichtu.ng (104) rotierend bewegt werden. Die Instrumentenspitze besteht aus mindestens 3 zueinander beweglichen Baugruppen. Eine erste Baugruppe (121) ist dabei um die Drehachse (111) in der Drehrichtung (105) kippbar zum Instrumentenschaft (120) beweglich angeordnet. Diese Baugruppe (121) trägt selbst zwei Baugruppen (122, 123), welche unabhängig voneinander kippbar um die Drehachse (112) in der Drehrichtung (106) angeordnet sind. Durch Drehbewegung der Baugruppe (122) relativ zur Baugruppe (123) wird der Winkel (107) zwischen den beiden Baugruppen (122, 123) um die Drehachse (112) verändert. Damit kann eine Greif-, Klemm- oder Schneidbewegung ausgeführt werden, je nachdem wie die Baugruppen (122, 123) mechanisch gestaltet sind.

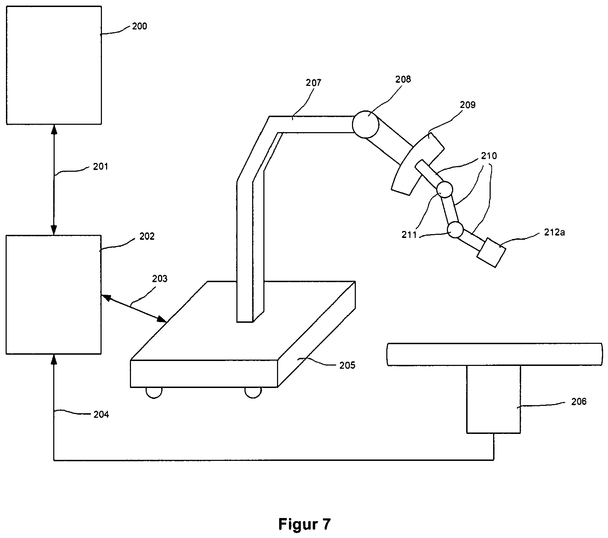

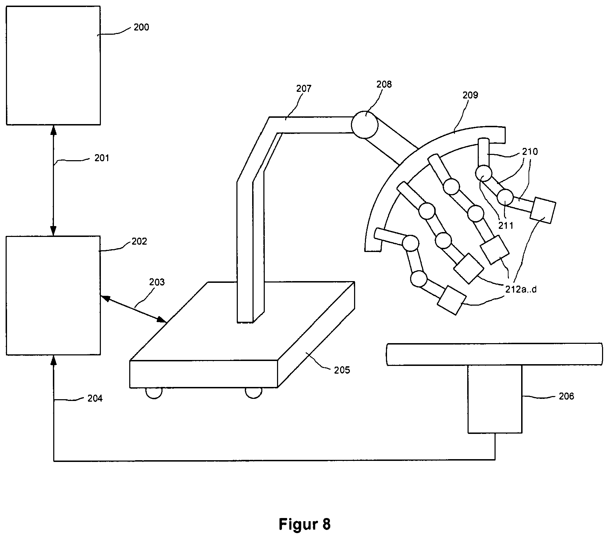

**Figur 7** und **Figur 8** zeigen Ausführungsformen des erfindungsgemäßen Robotersystems mit einem bzw. 4 Roboterarmen und mit einer (212a) bzw. 4 (212a-d) erfindungsgemäßen aktiven Positioniereinrichtungen. Die nachfolgenden Erläuterungen beziehen sich auf eine Ausführungsform mit einem Roboterarm gem. Figur 7. Die aktive Positioniereinrichtung (212a) ist über eine Vorpositionierungseinrichtung bestehend aus den Baugruppen 210 und 211 mit einer Bogenführung (209) verbunden. Die Vorpositionierungseinrichtung kann passiv, d.h. durch manuelle Verstellung oder vorzugsweise auch aktiv, d.h. durch Ausstattung der Gelenke (211) mit aktiven Stellantrieben realisiert sein. Die Vorpositionierungseinrichtung selbst wird mittels einer geeigneten Halterung, z.B. als Bogenführung (209) gehaltert. Diese Bogenführung (209) kann mittels des Gelenkes (208) zum Patienten positioniert werden. Der Ausleger (207) ist verbunden mit dem fahrbaren Trägersystem (205) und ermöglicht damit eine Positionierung des gesamten Trägersystems (bestehend aus 205, 207..212a) relativ zum OP-Tisch (206). Über die Bedien- und Anzeigeeinheit (200) wird dem Bediener der aktuelle Status der Vorpositioniereinrichtung (210, 211) ausgegeben. Über die Bedien- und Anzeigeeinheit (200) kann der Bediener Steuerbefehle eingeben, welche über eine geeignete Datenverbindung (201) an die Steuereinheit (202) und von dieser an die Aktive Positioniereinrichtung (212a), an die Vorpositioniereinrichtung (210, 211) sowie an die Bogenführung (209) zur weiteren Verarbeitung gesendet werden. Die Steuereinheit (202) ist über eine geeignete Datenverbindung (203) mit dem Trägersystem verbunden. Der OP-Tisch (206) kann steuerungstechnisch über die Datenverbindung (204) ebenfalls mit der Steuereinheit (202) verbunden sein, um bei einer Veränderung der OP-Tisch - Position, z.B. der Höhe, diese Positionsänderung in der Steuereinheit zu verarbeiten und eine aktive Nachführung der Aktiven Positioniereinrichtung (212a) über die Vorpositionierungseinrichtung (210, 211) und/oder die Lage der Bogenführung (209) zu erzielen. Damit können Veränderungen der Patientenposition aufgrund einer Positionsveränderung des OP-Tisches (206) aktiv ausgeglichen werden.

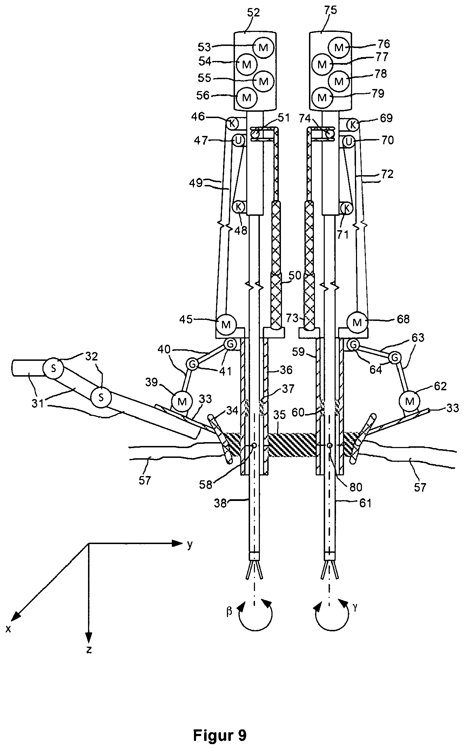

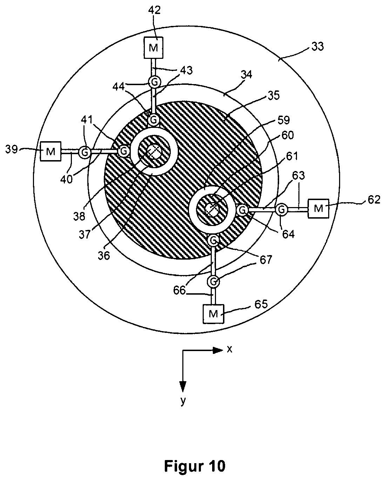

**Figur 9** **sowie** **Figur 10** zeigt eine erfindungsgemäße aktive Positioniereinrichtung für zwei chirurgische Instrumente an einem gemeinsamen Port, die an einem Roboterarm angebracht ist. Während eines minimal-invasiven, laparoskopischen Eingriffs kommen in der Regel 4 Operations-Instrumente zum Einsatz, hiervon 3 chirurgische Instrumente und 1 Kamera bzw. Endoskop, die über das Telemanipulatorsystem vom Operateur gesteuert werden. Erfindungsgemäß können daher 2 chirurgische Instrumente über einen gemeinsamen Port (single-Port) mittels zwei separaten Führungseinrichtungen in den Körper des Patienten eingeführt werden und mittels je einer aktiven Positioniereinrichtung pro chirurgischen Instrument voneinander unabhängig gesteuert werden. Es sind demnach vorzugsweise 2 Ausführungen eines aktiven Trokars für eine Single-Port-Zugang bzw. einer aktiven Positioniereinrichtung im System vorhanden. Es versteht sich jedoch, dass auch Ausführungsformen mit 1 oder mehr als 2 aktiven Trokaren für einen Single-Port-Zugang unter den Schutzbereich der Erfindung fallen. Jeder aktive Trokar wird über einen Roboterarm 31, welche mit Gelenken 32 versehen sein kann gewichtskraftfrei gelagert. Dieser Halterungsmechanismus ist demnach für jeden aktiven Trokar vorzusehen. Alle Halterungsmechanismen können an einem gemeinsamen Träger (siehe Figur 4), oder an getrennten Trägern befestigt werden. Eine Befestigung an getrennten Trägern kann beispielsweise sinnvoll sein, wenn die Anordnung der Trokare für den chirurgischen Eingriff dies erfordert.

Mit dem Roboterarm 31 ist eine Trägerplatte 33 des aktiven Trokars fest verbunden. Diese Trägerplatte 33 ist wiederum fest mit einer Porteinrichtung 34 verbunden. Die Porteinrichtung 34 ist seinerseits über ein Ausgleichselement 35 mit den Führungseinrichtungen 36 und 59 verbunden. Über das Ausgleichselement 35 ist eine Bewegung (Neigung) der Führungseinrichtungen 36 und 59 gegenüber der Porteinrichtung 34 möglich. Durch diese Bewegung wird die pivotale Bewegung der chirurgischen Instrumente 38 und 61 realisiert. Die Führungseinrichtungen 36 und 59 nehmen die chirurgischen Instrumente 38 und 61 auf. Über die Dichtungsringe 37 und 60 erfolgt eine gasdichte Abdichtung der chirurgischen Instrumente 38 und 61 gegenüber den Führungseinrichtungen 36 und 59. In der Laparoskopie wird der Bauchraum durch Einleitung eines Gases (Kohlendioxid, CO2) "aufgepumpt", um dem Operateur mehr Bewegungsfreiheit für den eigentlichen chirurgischen Eingriff zu ermöglichen. Damit das Gas nicht entweichen kann, ist die Abdichtung 37 bzw. 60 erforderlich.

Die Aktuatoren bzw. Stellantriebe 39, 42 sowie 62, 65 sind jeweils orthogonal zueinander angeordnet. Über Kugelhebelmechanismen 40, 41, 43, 44 sowie 63, 64, 66, 67 werden Kräfte auf das obere Ende der Führungseinrichtungen 36 bzw. 59 ausgeübt, so dass diese sich relativ zur Porteinrichtung 34 in 2 Achsen (x, y) unabhängig voneinander bewegen lassen.

Weitere Stellantriebe 45, 68 sind an den oberen Enden der Führungseinrichtungen 36 und 59 angebracht. Über Stellantriebsmechaniken bestehend aus Klemme 46, Umlenkrolle 47, Klemme 48 und entsprechende Seilzüge 49 wird die translatorische Bewegung des Instrumentes 38 in z-Richtung realisiert. Über Stellantriebsmechaniken bestehend aus Klemme 69, Umlenkrolle 70, Klemme 71 und entsprechende Seilzüge 72 wird die translatorische Bewegung des Instrumentes 61 in z-Richtung realisiert.

Ein Teleskopsystem 50 ist über die Halteeinrichtung 51 mit einer Instrumentenantriebseinheit 52 derart verbunden, das eine Rotationsbewegung β des chirurgischen Instrumentes 38 um die z-Achse verhindert wird. Die Rotationsbewegung β des chirurgischen Instruments 38 wird durch einen Rotationsaktuator 53, welcher mit dem Schaft des chirurgischen Instruments 38 verbunden ist, realisiert. Die Instrumentenaktuatoren 54, 55 und 56 realisieren die Bewegungen des chirurgischen Instruments 38 in den Freiheitsgraden 105, 106 und 107, siehe Figur 6.

Ein Teleskopsystem 73 ist über die Halteeinrichtung 74 mit einer Instrumentenantriebseinheit 75 derart verbunden, das eine Rotationsbewegung γ des chirurgischen Instrumentes 61 um die z-Achse verhindert wird. Die Rotationsbewegung γ des chirurgischen Instruments 61 wird durch einen Rotationsaktuator 76, welcher mit dem Schaft des chirurgischen Instruments 61 verbunden ist, realisiert. Die Instrumentenaktuatoren 77, 78 und 79 realisieren die Bewegungen des chirurgischen Instruments 61 in den Freiheitsgraden 105, 106 und 107, siehe Figur 6.

**Figur 10** zeigt eine Draufsicht der aktiven Positioniereinrichtung gemäß Figur 9, aus welcher ersichtlich ist, dass die beiden Führungseinrichtungen 36, 59 durch das gemeinsame Ausgleichselement 35 verlaufen. Insbesondere ist aus Figur 10 ersichtlich, dass die erste Verstelleinrichtung 39, 40, 41, 42, 43, 44 für die erste Führungseinrichtung 36 und die zweite Verstelleinrichtung 62, 63, 64, 65, 66, 67 der zweiten Führungseinrichtung 59 für die beiden chirurgischen Instrumente derart angeordnet sind, dass eine Behinderung bzw. Einschränkung der beiden jeweiligen Verstelleinrichtungen vermieden wird.

Insgesamt betrifft die vorliegende Erfindung somit eine aktive Positioniereinrichtung, bei welcher ein oder auch mehrere chirurgische Instrumente durch einen Trokar hindurch für die minimalinvasive Chirurgie verwendet werden können.

## Patentansprüche

1. Aktive Positioniereinrichtung eines chirurgischen Instruments zur Verwendung an einem Roboterarm umfassend
eine Trägerplatte (3, 33), welche mit einem Roboterarm (1, 31) verbindbar ist,
eine Porteinrichtung (4, 34), die an der Trägerplatte (3, 33) angeordnet ist und die für den Durchgang zum Inneren eines Körpers vorgesehen ist,
zumindest eine Führungseinrichtung (6, 36, 59) zur Durchführung eines chirurgischen Instruments (8, 38 61) in den Körper, wobei sich der Schaft des chirurgischen Instruments (8, 38, 61) durch die Führungseinrichtung (6, 36, 59) hindurch erstreckt und wobei die Führungseinrichtung (6, 36, 59) mit der Porteinrichtung (4, 34) über ein Ausgleichselement (5, 35) variabel verbunden ist, und
eine Verstelleinrichtung (9, 10, 11, 12, 13, 14, 39, 40, 41, 42, 43, 44, 62, 63, 64, 65, 66, 67) für die Führungseinrichtung (6, 36, 59) gegenüber der Porteinrichtung (4, 34), die zum einen an der Trägerplatte (3, 33) und/oder der Porteinrichtung (4, 34) und zum anderen an der Führungseinrichtung (6, 36, 59) derart angebracht ist, dass der Schaft des chirurgischen Instruments (8, 38, 61) um einen Pivot-Punkt (28) sowohl in x-Richtung als auch in die dazu orthogonale y-Richtung gegenüber der Ausgangslage bewegbar ist, in welcher die Längserstreckung des chirurgischen Instruments, welche die orthogonal zur x-Richtung und y-Richtung liegende z-Richtung definiert, parallel zur Längserstreckung der Porteinrichtung (4, 34) verläuft,
wobei die Verstelleinrichtung (9, 10, 11, 12, 13, 14, 39, 40, 41, 42, 43, 44, 62, 63, 64, 65, 66, 67) zwei ansteuerbare Aktuatoren (9, 12, 39, 42, 62, 65) aufweist, welche als zueinander orthogonal angeordnete Stellantriebe (9, 12, 39, 42, 62, 65) ausgeführt sind, wobei ein Kugelhebelmechanismus (10, 11, 13, 14, 40, 41, 43, 44, 63, 64, 66, 67) zwischen der Führungseinrichtung (6, 36, 59) und der Trägerplatte (3, 33) oder der Porteinrichtung (4, 34) derart vorgesehen ist, dass durch die Stellantriebe (9, 12, 39, 42) mittels des Kugelhebelmechanismus (10, 11, 13, 14, 40, 41, 43, 44) die Führungseinrichtung (6, 36) gegenüber der Ausgangslage in x-Richtung und y-Richtung unabhängig voneinander positionierbar ist.

2. Aktive Positioniereinrichtung nach Anspruch 1, **dadurch gekennzeichnet, dass** das Ausgleichselement (5, 35) in seiner Geometrie derart variierbar ist, dass ein frei wählbarer Winkel sowohl in x- als auch in y-Richtung zwischen der Porteinrichtung (4, 34) und der Führungseinrichtung (6, 36, 59) im Hinblick auf die zueinander kongruente Ausgangslage einstellbar ist, wobei das Ausgleichselement (5, 35) insbesondere aus einem elastischen Material ausgebildet ist.

3. Aktive Positioniereinrichtung gemäß einem der Ansprüche 1 oder 2, **dadurch gekennzeichnet, dass** an der Führungseinrichtung (6, 36, 59) eine Translationsverstelleinrichtung (15, 16, 17, 18, 19, 20, 45, 46, 47, 48, 49, 50, 68, 69, 70, 71, 72, 73) vorgesehen ist, die mit dem chirurgischen Instrument (8, 38, 61) derart verbunden ist, dass der Schaft des chirurgischen Instruments in z-Richtung bewegbar ist.

4. Aktive Positioniereinrichtung nach Anspruch 3, **dadurch gekennzeichnet, dass** die Translationsverstelleinrichtung (15, 16, 17, 18, 19, 20, 45, 46, 47, 48, 49, 50, 68, 69, 70, 71, 72, 73) mittels eines Teleskopsystems (20, 50, 73) und/oder Seilzugsystems (15, 16, 17, 18, 19, 45, 46, 47, 48, 49, 68, 69, 70, 71, 72) den Schaft des chirurgischen Instruments (8, 38, 61) in z-Richtung bewegt.

5. Aktive Positioniereinrichtung nach einem der Ansprüche 1 bis 4, umfassend weiter ein chirurgisches Instrument (8, 38, 61), **dadurch gekennzeichnet, dass** an dem chirurgischen Instrument (8, 38, 61) eine Instrumentenantriebseinheit (22, 52, 75) vorgesehen ist, welche einen Rotationsaktuator (23) umfasst, durch den der Schaft des chirurgischen Instruments (8) gegenüber der Ausgangslage um die z-Richtung drehbar variiert wird.

6. Aktive Positioniereinrichtung nach Anspruch 5, **dadurch gekennzeichnet, dass** die Instrumentenantriebseinheit (22, 52, 75) drei Instrumentenaktuatoren (24, 25, 26, 54, 55, 56, 77, 78, 79) aufweist, durch welche die am distalen Ende angebrachte Wirkeinheit des chirurgischen Instruments (8, 38, 61) in drei weiteren Freiheitsgraden variierbar ist.

7. Aktive Positioniereinrichtung nach Anspruch 5 oder 6, **dadurch gekennzeichnet, dass** die Instrumentenantriebseinheit (22, 52, 75) über eine Halteeinrichtung (21, 51, 78) an dem proximalen Ende des Teleskopsystems (20, 50, 73) angeordnet ist.

8. Aktive Positioniereinrichtung nach einem der Ansprüche 1 bis 7, **dadurch gekennzeichnet, dass** zwei Führungseinrichtungen (36, 59) zur Durchführung von zwei chirurgischen Instrumenten (38, 61) durch ein Ausgleichselement (35) vorgesehen sind, wobei die Verstelleinrichtung (39, 40, 41, 42, 43, 44) der ersten Führungseinrichtung (36) im Wesentlichen spiegelverkehrt im Hinblick auf die Längsachse der beiden Führungseinrichtungen (36, 59) zu der Verstelleinrichtung (62, 63, 64, 65, 66, 67) der anderen Führungseinrichtung (59) angeordnet ist.

9. Chirurgisches Robotersystem zur Durchführung von operativen Eingriffen am menschlichen Körper umfassend
eine Steuereinrichtung (200, 202), welche von einem Benutzer zur Durchführung des operativen Eingriffs bedienbar ist,
eine Trägerstruktur (207, 208, 209), an welcher zwei oder mehrere Roboterarme angebracht sind, welche durch die Steuereinrichtung bewegbar sind,
und wobei an zumindest einem Roboterarm eine aktive Positioniereinrichtung nach Anspruch 1 vorgesehen ist.

10. Chirurgisches Robotersystem nach Anspruch 9, **dadurch gekennzeichnet, dass** das Ausgleichselement (5, 35) in seiner Geometrie derart variierbar ist, dass ein frei wählbarer Winkel sowohl in x- als auch in y-Richtung zwischen der Porteinrichtung (4, 34) und der Führungseinrichtung (6, 36, 59) im Hinblick auf die zueinander kongruente Ausgangslage einstellbar ist, wobei das Ausgleichselement (5, 35) insbesondere aus einem elastischen Material ausgebildet ist.

11. Chirurgisches Robotersystem gemäß einem der Ansprüche 9 oder 10, **dadurch gekennzeichnet, dass** an der Führungseinrichtung (6, 36, 59) eine Translationsverstelleinrichtung (15, 16, 17, 18, 19, 20, 45, 46, 47, 48, 49, 50, 68, 69, 70, 71, 72, 73) vorgesehen ist, die mit dem chirurgischen Instrument (8, 38, 61) derart verbunden ist, dass der Schaft des chirurgischen Instruments in z-Richtung bewegbar ist.

12. Chirurgisches Robotersystem nach Anspruch 11, **dadurch gekennzeichnet, dass** die Translationsverstelleinrichtung (15, 16, 17, 18, 19, 20, 45, 46, 47, 48, 49, 50, 68, 69, 70, 71, 72, 73) mittels eines Teleskopsystems (20, 50, 73) und/oder Seilzugsystems (15, 16, 17, 18, 19, 45, 46, 47, 48, 49, 68, 69, 70, 71, 72) den Schaft des chirurgischen Instruments (8, 38, 61) in z-Richtung bewegt.

13. Chirurgisches Robotersystem nach einem der Ansprüche 9 bis 12, umfassend weiter ein chirurgisches Instrument (8, 38, 61), **dadurch gekennzeichnet, dass** an dem chirurgischen Instrument (8, 38, 61) eine Instrumentenantriebseinheit (22, 52, 75) vorgesehen ist, welche einen Rotationsaktuator (23) umfasst, durch den der Schaft des chirurgischen Instruments (8) gegenüber der Ausgangslage um die z-Richtung drehbar variiert wird.

14. Chirurgisches Robotersystem nach Anspruch 13, **dadurch gekennzeichnet, dass** die Instrumentenantriebseinheit (22, 52, 75) drei Instrumentenaktuatoren (24, 25, 26, 54, 55, 56, 77, 78, 79) aufweist, durch welche die am distalen Ende angebrachte Wirkeinheit des chirurgischen Instruments (8, 38, 61) in drei weiteren Freiheitsgraden variierbar ist.

15. Chirurgisches Robotersystem nach Anspruch 13 oder 14, **dadurch gekennzeichnet, dass** die Instrumentenantriebseinheit (22, 52, 75) über eine Halteeinrichtung (21, 51, 78) an dem proximalen Ende des Teleskopsystems (20, 50, 73) angeordnet ist.

16. Chirurgisches Robotersystem nach einem der Ansprüche 9 bis 15, **dadurch gekennzeichnet, dass** zwei Führungseinrichtungen (36, 59) zur Durchführung von zwei chirurgischen Instrumenten (38, 61) durch ein Ausgleichselement (35) vorgesehen sind, wobei die Verstelleinrichtung (39, 40, 41, 42, 43, 44) der ersten Führungseinrichtung (36) im Wesentlichen spiegelverkehrt im Hinblick auf die Längsachse der beiden Führungseinrichtungen (36, 59) zu der Verstelleinrichtung (62, 63, 64, 65, 66, 67) der anderen Führungseinrichtung (59) angeordnet ist.

## Claims

1. Active positioning device of a surgical instrument for use on a robotic arm comprising
a support plate (3, 33) which can be connected to a robotic arm (1, 31),
a port device (4, 34) which is arranged on the support plate (3, 33) and which is provided for the passage to the inside of a body,
at least one guide device (6, 36, 59) for guiding a surgical instrument (8, 38, 61) through into the body, wherein the shaft of the surgical instrument (8, 38, 61) extends through the guide device (6, 36, 59) and wherein the guide device (6, 36, 59) is variably connected to the port device (4, 34) via a compensating element (5, 35), and
an adjustment device (9, 10, 11, 12, 13, 14, 39, 40, 41, 42, 43, 44, 62, 63, 64, 65, 66, 67) for the guide device (6, 36, 59) relative to the port device (4, 34), which is attached on the one hand to the support plate (3, 33) and/or the port device (4, 34) and on the other hand to the guide device (6, 36, 59) such that the shaft of the surgical instrument (8, 38, 61) is movable about a pivot point (28) both in the x-direction and in the y-direction, which is orthogonal thereto, relative to the starting position in which the longitudinal extension of the surgical instrument, which defines the z-direction lying orthogonal to the x-direction and y-direction, runs parallel to the longitudinal extension of the port device (4, 34),
wherein the adjustment device (9, 10, 11, 12, 13, 14, 39, 40, 41, 42, 43, 44, 62, 63, 64, 65, 66, 67) has two controllable actuators (9, 12, 39, 42, 62, 65) which are designed as actuating drives (9, 12, 39, 42, 62, 65) arranged orthogonal to each other, wherein a ball lever mechanism (10, 11, 13, 14, 40, 41, 43, 44, 63, 64, 66, 67) is provided between the guide device (6, 36, 59) and the support plate (3, 33) or the port device (4, 34) such that the guide device (6, 36) can be positioned relative to the starting position in the x-direction and y-direction independently of each other by the actuating drives (9, 12, 39, 42) by means of the ball lever mechanism (10, 11, 13, 14, 40, 41, 43, 44).

2. Active positioning device according to claim 1, **characterized in that** the geometry of the compensating element (5, 35) is variable such that a freely selectable angle can be set, both in the x-direction and in the y-direction, between the port device (4, 34) and the guide device (6, 36, 59) with regard to the mutually congruent starting position, wherein the compensating element (5, 35) is formed in particular of an elastic material.

3. Active positioning device according to one of claims 1 or 2, **characterized in that** a translational adjustment device (15, 16, 17, 18, 19, 20, 45, 46, 47, 48, 49, 50, 68, 69, 70, 71, 72, 73) which is connected to the surgical instrument (8, 38, 61) such that the shaft of the surgical instrument is movable in the z-direction is provided on the guide device (6, 36, 59).

4. Active positioning device according to claim 3, **characterized in that** the translational adjustment device (15, 16, 17, 18, 19, 20, 45, 46, 47, 48, 49, 50, 68, 69, 70, 71, 72, 73) moves the shaft of the surgical instrument (8, 38, 61) in the z-direction by means of a telescopic system (20, 50, 73) and/or cable pull system (15, 16, 17, 18, 19, 45, 46, 47, 48, 49, 68, 69, 70, 71, 72).

5. Active positioning device according to one of claims 1 to 4, further comprising a surgical instrument (8, 38, 61), **characterized in that** an instrument drive unit (22, 52, 75) which comprises a rotary actuator (23) by which the shaft of the surgical instrument (8) is rotatably varied about the z-direction relative to the starting position is provided on the surgical instrument (8, 38, 61).

6. Active positioning device according to claim 5, **characterized in that** the instrument drive unit (22, 52, 75) has three instrument actuators (24, 25, 26, 54, 55, 56, 77, 78, 79) by which the operative unit of the surgical instrument (8, 38, 61) attached to the distal end is variable in three further degrees of freedom.

7. Active positioning device according to claim 5 or 6, **characterized in that** the instrument drive unit (22, 52, 75) is arranged, via a holding device (21, 51, 78), at the proximal end of the telescopic system (20, 50, 73).

8. Active positioning device according to one of claims 1 to 7, **characterized in that** two guide devices (36, 59) are provided for guiding two surgical instruments (38, 61) through a compensating element (35), wherein the adjustment device (39, 40, 41, 42, 43, 44) of the first guide device (36) is arranged substantially mirror-inverted with regard to the longitudinal axis of the two guide devices (36, 59) relative to the adjustment device (62, 63, 64, 65, 66, 67) of the other guide device (59).

9. Robotic surgical system for carrying out surgical procedures on the human body comprising
a control device (200, 202) which can be operated by a user to carry out the surgical procedure,
a support structure (207, 208, 209) to which two or more robotic arms are attached which can be moved by the control device, and wherein an active positioning device according to claim 1 is provided on at least one robotic arm.

10. Robotic surgical system according to claim 9, **characterized in that** the geometry of the compensating element (5, 35) is variable such that a freely selectable angle can be set, both in the x-direction and in the y-direction, between the port device (4, 34) and the guide device (6, 36, 59) with regard to the mutually congruent starting position, wherein the compensating element (5, 35) is formed in particular of an elastic material.

11. Robotic surgical system according to one of claims 9 or 10, **characterized in that** a translational adjustment device (15, 16, 17, 18, 19, 20, 45, 46, 47, 48, 49, 50, 68, 69, 70, 71, 72, 73) which is connected to the surgical instrument (8, 38, 61) such that the shaft of the surgical instrument is movable in the z-direction is provided on the guide device (6, 36, 59).

12. Robotic surgical system according to claim 11, **characterized in that** the translational adjustment device (15, 16, 17, 18, 19, 20, 45, 46, 47, 48, 49, 50, 68, 69, 70, 71, 72, 73) moves the shaft of the surgical instrument (8, 38, 61) in the z-direction by means of a telescopic system (20, 50, 73) and/or cable pull system (15, 16, 17, 18, 19, 45, 46, 47, 48, 49, 68, 69, 70, 71, 72).

13. Robotic surgical system according to one of claims 9 to 12, further comprising a surgical instrument (8, 38, 61), **characterized in that** an instrument drive unit (22, 52, 75) which comprises a rotary actuator (23) by which the shaft of the surgical instrument (8) is rotatably varied about the z-direction relative to the starting position is provided on the surgical instrument (8, 38, 61).

14. Robotic surgical system according to claim 13, **characterized in that** the instrument drive unit (22, 52, 75) has three instrument actuators (24, 25, 26, 54, 55, 56, 77, 78, 79) by which the operative unit of the surgical instrument (8, 38, 61) attached to the distal end is variable in three further degrees of freedom.

15. Robotic surgical system according to claim 13 or 14, **characterized in that** the instrument drive unit (22, 52, 75) is arranged, via a holding device (21, 51, 78), at the proximal end of the telescopic system (20, 50, 73).

16. Robotic surgical system according to one of claims 9 to 15, **characterized in that** two guide devices (36, 59) are provided for guiding two surgical instruments (38, 61) through a compensating element (35), wherein the adjustment device (39, 40, 41, 42, 43, 44) of the first guide device (36) is arranged substantially mirror-inverted with regard to the longitudinal axis of the two guide devices (36, 59) relative to the adjustment device (62, 63, 64, 65, 66, 67) of the other guide device (59).

## Revendications

1. Dispositif de positionnement actif d'un instrument chirurgical destiné à être utilisé sur un bras de robot, comprenant
une plaque de support (3, 33), qui peut être reliée à un bras de robot (1, 31),
un dispositif d'accès (4, 34), qui est disposé sur la plaque de support (3, 33) et qui est prévu pour le passage à l'intérieur d'un corps,
au moins un dispositif de guidage (6, 36, 59) destiné à faire passer un instrument chirurgical (8, 38, 61) dans le corps, la tige de l'instrument chirurgical (8, 38, 61) s'étendant à travers le dispositif de guidage (6, 36, 59) et le dispositif de guidage (6, 36, 59) étant relié de manière variable au dispositif d'accès (4, 34) par le biais d'un élément de compensation (5, 35), et
un dispositif de réglage (9, 10, 11, 12, 13, 14, 39, 40, 41, 42, 43, 44, 62, 63, 64, 65, 66, 67) pour le dispositif de guidage (6, 36, 59) par rapport au dispositif d'accès (4, 34), qui est monté d'une part sur la plaque de support (3, 33) et/ou le dispositif d'accès (4, 34) et d'autre part sur le dispositif de guidage (6, 36, 59) de telle manière que la tige de l'instrument chirurgical (8, 38, 61) est mobile autour d'un point de pivot (28) aussi bien dans la direction x que dans la direction y perpendiculaire à celle-ci par rapport à la position initiale, dans laquelle l'extension longitudinale de l'instrument chirurgical, qui définit la direction z perpendiculaire à la direction x et la direction y, s'étend parallèlement à l'extension longitudinale du dispositif d'accès (4, 34),
le dispositif de réglage (9, 10, 11, 12, 13, 14, 39, 40, 41, 42, 43, 44, 62, 63, 64, 65, 66, 67) comportant deux actionneurs (9, 12, 39, 42, 62, 65) pouvant être commandés, lesquels sont réalisés sous la forme d'organes moteurs (9, 12, 39, 42, 62, 65) disposés perpendiculairement l'un à l'autre, un mécanisme de levier à rotule (10, 11, 13, 14, 40, 41, 43, 44, 63, 64, 66, 67) étant prévu entre le dispositif de guidage (6, 36, 59) et la plaque de support (3, 33) ou le dispositif d'accès (4, 34) de telle manière que le dispositif de guidage (6, 36) peut être positionné par les organes moteurs (9, 12, 39, 42) au moyen du mécanisme de levier à rotule (10, 11, 13, 14, 40, 41, 43, 44) dans la direction x et la direction y indépendamment l'une de l'autre par rapport à la position initiale.

2. Dispositif de positionnement actif selon la revendication 1, **caractérisé en ce que** l'élément de compensation (5, 35) est à géométrie variable de telle manière qu'un angle pouvant être choisi librement peut être réglé aussi bien dans la direction x que dans la direction y entre le dispositif d'accès (4, 34) et le dispositif de guidage (6, 36, 59) en ce qui concerne la position initiale, dans laquelle ils sont congruents l'un par rapport à l'autre, l'élément de compensation (5, 35) étant réalisé en particulier en un matériau élastique.

3. Dispositif de positionnement actif selon l'une des revendications 1 ou 2, **caractérisé en ce qu'**un dispositif de réglage en translation (15, 16, 17, 18, 19, 20, 45, 46, 47, 48, 49, 50, 68, 69, 70, 71, 72, 73) est prévu sur le dispositif de guidage (6, 36, 59), ledit dispositif de réglage en translation étant relié à l'instrument chirurgical (8, 38, 61) de telle manière que la tige de l'instrument chirurgical peut être déplacée dans la direction z.

4. Dispositif de positionnement actif selon la revendication 3, **caractérisé en ce que** le dispositif de réglage en translation (15, 16, 17, 18, 19, 20, 45, 46, 47, 48, 49, 50, 68, 69, 70, 71, 72, 73) déplace la tige de l'instrument chirurgical (8, 38, 61) dans la direction z au moyen d'un système télescopique (20, 50, 73) et/ou d'un système de transmission par câble (15, 16, 17, 18, 19, 45, 46, 47, 48, 49, 68, 69, 70, 71, 72).

5. Dispositif de positionnement actif selon l'une des revendications 1 à 4, comprenant en outre un instrument chirurgical (8, 38, 61), **caractérisé en ce qu'**une unité d'entraînement d'instrument (22, 52, 75) est prévue sur l'instrument chirurgical (8, 38, 61), laquelle comprend un actionneur rotatif (23), par lequel on fait varier la tige de l'instrument chirurgical (8) de manière rotative autour de la direction z par rapport à la position initiale.

6. Dispositif de positionnement actif selon la revendication 5, **caractérisé en ce que** l'unité d'entraînement d'instrument (22, 52, 75) comporte trois actionneurs d'instrument (24, 25, 26, 54, 55, 56, 77, 78, 79), par lesquels on peut faire varier l'unité d'action de l'instrument chirurgical (8, 38, 61) montée sur l'extrémité distale selon trois autres degrés de liberté.

7. Dispositif de positionnement actif selon la revendication 5 ou 6, **caractérisé en ce que** l'unité d'entraînement d'instrument (22, 52, 75) est disposée sur l'extrémité proximale du système télescopique (20, 50, 73) par le biais d'un dispositif de maintien (21, 51, 78).

8. Dispositif de positionnement actif selon l'une des revendications 1 à 7, **caractérisé en ce que** deux dispositifs de guidage (36, 59) sont prévus pour faire passer deux instruments chirurgicaux (38, 61) à travers un élément de compensation (35), le dispositif de réglage (39, 40, 41, 42, 43, 44) du premier dispositif de guidage (36) et le dispositif de réglage (62, 63, 64, 65, 66, 67) de l'autre dispositif de guidage (59) étant disposés de manière sensiblement symétrique par rapport à l'axe longitudinal des deux dispositifs de guidage (36, 59).

9. Système robotique chirurgical destiné à effectuer des interventions opératoires sur le corps humain, comprenant
un dispositif de commande (200, 202), qui peut être manoeuvré par un utilisateur pour effectuer l'intervention opératoire,
une structure de support (207, 208, 209), sur laquelle deux bras de robot ou plus sont montés, qui peuvent être déplacés par le dispositif de commande, et un dispositif de positionnement actif selon la revendication 1 étant prévu sur au moins un bras de robot.

10. Système robotique chirurgical selon la revendication 9, **caractérisé en ce que** l'élément de compensation (5, 35) est à géométrie variable de telle manière qu'un angle pouvant être choisi librement peut être réglé aussi bien dans la direction x que dans la direction y entre le dispositif d'accès (4, 34) et le dispositif de guidage (6, 36, 59) en ce qui concerne la position initiale dans laquelle ils sont congruents l'un par rapport à l'autre, l'élément de compensation (5, 35) étant réalisé en particulier en un matériau élastique.

11. Système robotique chirurgical selon l'une des revendications 9 ou 10, **caractérisé en ce qu'**un dispositif de réglage en translation (15, 16, 17, 18, 19, 20, 45, 46, 47, 48, 49, 50, 68, 69, 70, 71, 72, 73) est prévu sur le dispositif de guidage (6, 36, 59), ledit dispositif de réglage en translation étant relié à l'instrument chirurgical (8, 38, 61) de telle manière que la tige de l'instrument chirurgical peut être déplacée dans la direction z.

12. Système robotique chirurgical selon la revendication 11, **caractérisé en ce que** le dispositif de réglage en translation (15, 16, 17, 18, 19, 20, 45, 46, 47, 48, 49, 50, 68, 69, 70, 71, 72, 73) déplace la tige de l'instrument chirurgical (8, 38, 61) dans la direction z au moyen d'un système télescopique (20, 50, 73) et/ou d'un système de transmission par câble (15, 16, 17, 18, 19, 45, 46, 47, 48, 49, 68, 69, 70, 71, 72).

13. Système robotique chirurgical selon l'une des revendications 9 à 12, comprenant en outre un instrument chirurgical (8, 38, 61), **caractérisé en ce qu'**une unité d'entraînement d'instrument (22, 52, 75) est prévue sur l'instrument chirurgical (8, 38, 61), laquelle comprend un actionneur rotatif (23), par lequel on fait varier la tige de l'instrument chirurgical (8) de manière rotative autour de la direction z par rapport à la position initiale.

14. Système robotique chirurgical selon la revendication 13, **caractérisé en ce que** l'unité d'entraînement d'instrument (22, 52, 75) comporte trois actionneurs d'instrument (24, 25, 26, 54, 55, 56, 77, 78, 79), par lesquels on peut faire varier l'unité d'action de l'instrument chirurgical (8, 38, 61) montée sur l'extrémité distale selon trois autres degrés de liberté.

15. Système robotique chirurgical selon la revendication 13 ou 14, **caractérisé en ce que** l'unité d'entraînement d'instrument (22, 52, 75) est disposée sur l'extrémité proximale du système télescopique (20, 50, 73) par le biais d'un dispositif de maintien (21, 51, 78).

16. Système robotique chirurgical selon l'une des revendications 9 à 15, **caractérisé en ce que** deux dispositifs de guidage (36, 59) sont prévus pour faire passer deux instruments chirurgicaux (38, 61) à travers un élément de compensation (35), le dispositif de réglage (39, 40, 41, 42, 43, 44) du premier dispositif de guidage (36) et le dispositif de réglage (62, 63, 64, 65, 66, 67) de l'autre dispositif de guidage (59) étant disposés de manière sensiblement symétrique par rapport à l'axe longitudinal des deux dispositifs de guidage (36, 59).
